# EUROPEAN PATENT SPECIFICATION

(11) **EP 0 426 948 B1**
(45) Date of publication and mention of the grant of the patent: **08.05.1996**
(21) Application number: 90112833.0
(22) Date of filing: 05.07.1990
(51) Int. Cl.: C07D 207/325, A01N 43/34

(54) **Arylpyrrole insecticidal acaricidal and nematicidal agents and methods**
Arylpyrrol enthaltende insekticidale, acaricidale und nematicidale Mittel sowie Verfahren zu ihrer Herstellung
Agents nématocides, acaricides et insecticides comprenant de l'arylpyrrole et procédés pour leur préparation

(30) Priority: 11.08.1989 US 392495
(43) Date of publication of application: 15.05.1991
(73) Proprietor: AMERICAN CYANAMID COMPANY, Wayne, NJ 07470-8426 (US)
(72) Inventor: Brown, Dale Gordon, HOPEWELL, NEW JERSEY (US); Siddens, Jack Kenneth, . (US); Diehl, Robert Eugene, Lawrenceville, New Jersey (US); Wright, Donald Perry, Jr., Pennington, New Jersey (US)
(74) Representative: Wächtershäuser, Günter, Prof. Dr.

(56) References cited:
- EP-A- 0 182 737
- GB-A- 1 231 804
- US-A- 3 487 089

## Description

The present invention is directed to certain novel arylpyrrole compounds that are highly effective insecticidal, acaricidal and nematicidal agents useful for the control of insect, acarid and nematode pests and for protecting agronomic crops, both growing and harvested, against the ravages of said pests. The present invention is also directed to methods for preparing the arylpyrrole compounds.

EP-A-0 182 737 discloses 3-phenylpyrrol derivatives, a process for their preparation and use thereof as pesticides. The compounds of the present invention are not described in this document.

The novel arylpyrrole compounds of the present invention have the structural formula illustrated as formula I: 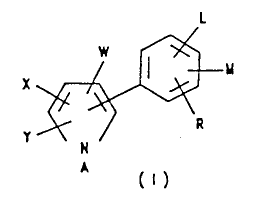
wherein
- X is: H, F, Cl, Br, I, or CF₃;
- Y is: H, F, Cl, Br, I, CF₃ or CN;
- W is: CN or NO₂;
- A is: C₁-C₄ alkyl substituted with four halogen atoms, one cyano, one C₁-C₄ alkoxy group substituted with one to three halogen atoms or two alkoxy groups optionally substituted with one to three halogen atoms, one C₁-C₄ carbalkoxy, one C₁-C₆ alkylcarbonyloxy, one C₂-C₆ alkenylcarbonyloxy, one benzenecarbonyloxy or chloro, dichloro, or methyl-substitutedbenzenecarbonyloxy;
- L is: H, F, Cl or Br;
- M and R are: each independently H, C₁-C₃ alkyl, C₁-C₃ alkoxy, C₁-C₃ alkylthio, C₁-C₃ alkylsulfinyl, C₁-C₃ alkylsulfonyl, cyano, F, Cl, Br, I, nitro, CF₃, R₁CF₂Z, R₂CO or NR₃R₄ and when M and R are on adjacent positions and taken with the carbon atoms to which they are attached they may form a ring in which MR represents the structure:
-OCH₂O- , -OCF₂O- or 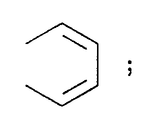
- Z is: S(O)n or O;
- R₁ is: H, F, CHF₂, CHFC1, or CF₃;
- R₂ is: C₁-C₃ alkyl, C₁-C₃ alkoxy, or NR₃R₄;
- R₃ is: H or C₁-C₃ alkyl;
- R₄ is: H, C₁-C₃ alkyl or R₅CO;
- R₅ is: H or C₁-C₃ alkyl and
- n is: an integer of 0, 1 or 2;
provided that when X and Y are H and A is C₁-C₄ alkyl substituted with one cyano, or one C₁₋₄ carbalkoxy, then the 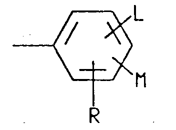
substituent must be fixed to one of the two positions on the pyrrole ring adjacent to the nitrogen atom.

A preferred group of novel arylpyrroles of the present invention are illustrated by formula II: 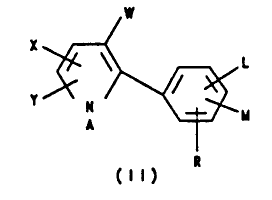
wherein A, L, M, R, W, X and Y are as described above.

Another preferred group of novel arylpyrroles of this invention are represented by formula III: 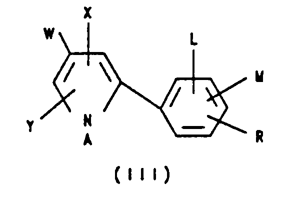
wherein A, L, M, R, W, X and Y are as described above.

Another group of preferred arylpyrroles of the invention are depicted by formula IV: 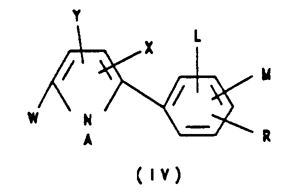
wherein A, L, M, R, W, X and Y are as described above.

Yet another group of preferred arylpyrroles of this invention are delineated by formula V: 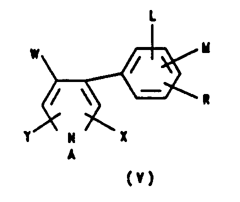
wherein A, L, M R, W, X and Y are as described above; and still other preferred arylpyrroles of the invention are depicted by formulas VI and VII: 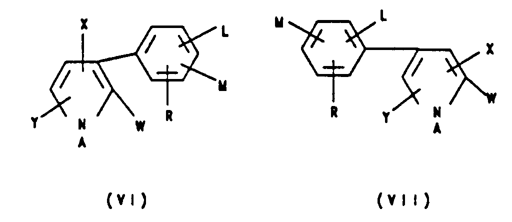
wherein A, L, M, R, W, X and Y are as described above.

Preferred formula I arylpyrroles of the invention are those in which
- W is: CN or NO₂;
- L is: hydrogen;
- X and Y are: each Cl, Br or CF₃;
- M is: H, F, Cl or Br and
- R is: F, Cl, Br, CF₃ or OCF₃.

Preferred formula II compounds which are especially effective as insecticidal, acaricidal and/or nematicidal agents are those in which
- L is: hydrogen;
- M is: hydrogen, F, Cl or Br;
- R is: F, Cl, Br, CF₃ or OCF₃;
- W is: CN and
- X and Y are: each independently Cl, Br or CF₃.

Other formula II compounds that are highly effective as insecticidal, acaricidal and/or nematicidal agents are those in which
- L is: hydrogen;
- M is: hydrogen, F, Cl or Br;
- R is: F, Cl, Br, CF₃ or OCF₃;
- W is: NO₂;
- X is: Cl, Br or CF₃ and
- Y is: H, Cl, Br or CF₃.

Illustrative of some of insecticidal, acaricidal and nematicidal arylpyrroles of the present invention are:
[2,3-dichloro-4-cyano-5-(3,4-dichlorophenyl)pyrrol-1-yl]-pivalic acid, methyl ester, mp 143°-145°C;
4,5-dichloro-1-(hydroxymethyl)-2-α,α,α-trifluoro-p-tolyl)pyrrole-3-carbonitrile, acetate (ester);
4-bromo-2-(p-chlorophenyl-1-(hydroxymethyl)-5-(trifluoromethyl)pyrrole-3-carbonitrile, pivalate (ester), mp 127.5°C;
4-bromo-3-(3,4-dichlorophenyl)-1-(hydroxymethyl)-5-(trifluoromethyl)pyrrole-2-carbonitrile, pivalate ester, mp 93°-94°C;
3,4-dibromo-5-(p-chlorophenyl)-1-(hydroxymethyl)pyrrole-2-carbonitrile, pivalate (ester), mp 130°-132°C;
[2,3-dichloro-4-cyano-5-(α,α,α-trifluoro-p-tolyl)pyrrol-1-yl]pivalic acid, methyl ester, mp 153°-157°C;
3-bromo-5-cyano-4-(3,4-dichlorophenyl)-2-(trifluoromethyl)pyrrole-1-acetonitrile, mp 95°-97°C;
2,3-dichloro-4-cyano-5-(α,α,α-trifluoro-p-tolyl)pyrrol-1-acetonitrite, mp 137.5°-139°C;
3-bromo-5-(p-chlorophenyl)-4-cyano-2-(trifluoromethyl)pyrrole-1-acetonitrile, mp 142°-144°C;
2,3-dichloro-4-cyano-(3,4-dichlorophenyl)pyrrole-1-acetonitrile, mp 197°-199°C;
[2-(3,4-dichlorophenyl)3-nitro-5-(trifluoromethyl)pyrrol-1-yl]benzoic acid, methyl ester;
[2,3-dichloro-4-cyano-5-(3,4-diclorophenyl)pyrrol-1-yl]-benzoic acid, methyl ester;
3-bromo-5-(p-bromophenyl)-4-cyano-2-(trifluoromethyl)pyrrole-1-acetonitrile, mp 110.5°-113°C;
[3,bromo-5-chlro-4-cyano-2-(3,4-dichlorophenyl)pyrrol-1-yl]pivalic acid, methyl ester, mp 119°-122°C;
[2,3-dichloro-4-cyano-5-(α,α,α-trifluoro-p-tolyl)pyrrol-1-yl]p-chlorobenzoic acid, methyl ester;
4-bromo-2-(p-chlorophenyl)-1-hydroxymethyl)-5-trifluoromethyl)pyrrole-3-carbonitrile, p-chlorobenzoate (ester);
2,3-dichloro-5-(p-chlorophenyl)-4-cyano-pyrrole-1-acetonitrile, mp 175°-177°C;
[2-(3,4-dichlorophenyl)-3-nitro-5-(trifluoromethyl)pyrrol-1-yl]pivalic acid, methyl ester, mp 116°-119°C;
3-bromo-2-(p-chlorophenyl)-1-[2,2,2-trifluoroethoxy)methyl]-5-(trifluoromethyl)pyrrole-3-carbonitrile and
3-bromo-5-chloro-2-(p-chlorophenyl)-4-cyano-pyrrole-3-carbonitrile, mp 144°-150°C.

Methods of preparation of arylpyrrole compounds of formula I wherein A is hyrdogen are described in co-pending European Patent Application Publication Number 0 347 488 (application No. 88 112 810.2).

Preparation of N-substituted formula I arylpyrroles can be achieved by reaction of the appropriately substituted formula I arylpyrrole, wherein A is hydrogen and L, M, R, W, X and Y are as described above, with an appropriate alkylating agent and a suitable base, for example, a chloromethyl C₁-C₄-haloalkyl ether and potassium hydride This reaction provides an arylpyrrole having the same substituents as the starting material, but in addition is substituted on the nitrogen with C₁-C₄ haloalkoxymethyl. In a similar reaction α-bromoacetonitrile is substituted for the chloromethyl C₁-C₄ haloalkyl ether and yields the formula I arylpyrrole with an acetonitrile substituent on the nitrogen. The reactions may be illustrated as follows: 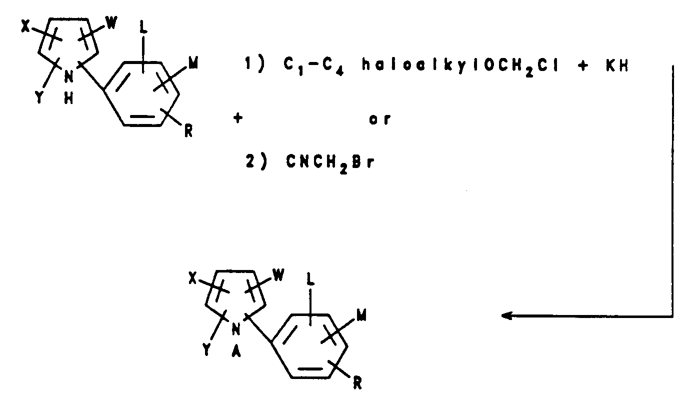
wherein L, M, R, W, X and Y are as described for formula I above and A is 1) C₁-C₄ haloalkoxymethyl or 2) CH₂CN.

The examples provided by way of illustration below utilize the scheme illustrated above and provide a means for preparing other compounds of the invention which are not specifically described herein.

The arylpyrroles of the present invention are effective for controlling insects, acarina and nematodes. These compounds are also effective for protecting growing or harvested crops from attack by the above-said pests.

In practice generally about 10 ppm to about 10,000 ppm and preferably 100 to about 5000 ppm, of the formula I arylpyrrole, which encompasses all of the arylpyrrole isomers of formulas II, III, IV, V, VI and VII, dispersed in water or other inexpensive liquid carrier is effective when applied to the plants, the crops or the soil in which said crops are growing to protect said crops from attack by insects, acarina and/or nematodes. These compounds are also useful for protecting turf grass from attack by pests such as grubs, chinch bugs and the like.

The formula I arylpyrroles of this invention are also effective for controlling insects, nematodes and acarina, when applied to the foliage of plants and/or to the soil or water in which said plants are growing in sufficient amount to provide a rate of from about 0.100 kg/ha to about 4.0 kg/ha of active ingredient. Obviously higher rates of application of the formula I arylpyrroles may be used to protect crops from attack by insects, nematodes and acarina, however, higher rates of application are generally unnecessary and wasteful.

While the arylpyrroles of this invention are effective for controlling insects, nematodes and acarina when employed alone, they may be used in combination with other biological chemicals, including other insecticides, nematicides and acaricides. For example, the arylpyrroles of this invention may be used effectively in conjunction or combination with phosphates, carbamates, pyrethroids, formamidines, chlorinated hydrocarbons, halobenzoylureas and the like.

Advantageously, the above-said arylpyrroles may be formulated into dry compacted granules, flowable compositions, granular formulations, wettable powders, dusts, dust concentrates, microemulsions and the like, all of which lend themselves to soil, water and/or foliage application and provide the requisite plant protection. Such formulations include the compounds of the invention admixed with inert, pharmacologically-acceptable solid or liquid diluents.

For example, wettable powders, dusts and dust concentrate formulations of the invention can be prepared by grinding together about 3% to 20%, by weight, of the formula I arylpyrrole compound, with about 3% to 20% by weight of a solid anionic surfactant. One suitable anionic surfactant is a dioctyl ester of sodium sulfosuccinic acid, specifically Aerosol OTB® surfactant marketed by the American Cyanamid Company. About 60% to 94%, by weight, of an inert solid diluent, such as montmorillonite, attapulgite, chalk, talc, kaolin, diatomaceous earth, limestone, silicates or the like also is used in such formulations.

Compacted granules especially useful for soil or water application can be prepared by grinding together in about equal parts, usually about 3 to 20 parts, of the arylpyrrole and a solid surfactant, with about 60 to 94 parts of gypsum. Thereafter, the mixture is compacted into small granular particles, about 24/48 mesh or larger.

Other suitable solid surfactants useful in the present formulations include not only the anionic dioctyl ester of sodium sulfosuccinic acid but also nonionic block copolymers of ethylene oxide and propylene oxide. Such block copolymers are marketed by BASF Wyandotte Corporation as Pluronic 10R8®, 17R8®, 25R8®, F38®, F68®, F77®, or F87®, and are especially effective for the preparation of compacted granules.

In addition to the powders and concentrate formulations described hereinabove, wettable powders and flowables may be used because they may be dispersed in water. Preferably, such flowables will be applied at the locus with the aqueous compositions being sprayed on the foliage of plants to be protected. These sprays also may be applied to the breeding ground, food supply or habitat of the insects and acarina sought to be controlled.

Where solid formulations of the compounds of this invention are to be used in combination treatments with other pesticidal agents, the formulations can be applied as an admixture of the components or may be applied sequentially.

Similarly, liquid formulations of the aryl-pyrrole in combination with other pesticidal agents may be tank mixed or may be applied separately, sequentially, as liquid sprays. Liquid spray formulations of the compounds of the invention should contain about 0.001% to about 0.1% by weight of the active arylpyrrole.

The following examples are presented as illustrations of the present invention.

### EXAMPLE 1

### 3-Bromo-2-(p-chlorophenyl)-1-[2,2,2-trifluoroethoxy)methyl]-5-(trifluoromethyl)pyrrole-3-carbonitrile

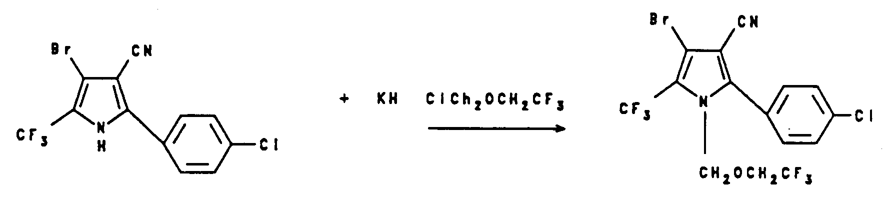

A sample of 3-bromo-2-(p-chlorophenyl)-5-(trifluoromethyl)pyrrole-3-carbonitrile (1.0g, 0.003 mole) is dissolved in dry tetrahydrofuran and added to a tetrahydofuran dispersion of potassium hydride (1.25 equivalents). The reaction mixture is stirred for 10 minutes, treated with chloromethyl(trifluoromethyl) ether (0.54g, 0.0036 mol), stirred for about 48 hours at room temperature and then poured into a large volume of water and extracted with ether. The ether extract is washed with brine, dried (MgSO₄) and concentrated in vacuo to give a residue. After flash chromatography (silica and 1:1 methylene chloride:hexanes) the title product is obtained as a white solid 0.90g (68%y), mp 124.6°-125.3°C.

Following the procedure described above, but using the appropriately substituted phenylpyrrole-3-carbonitrile, or 3-nitro-2-(substituted)phenylpyrrole and the appropriate alkylating agent, the compounds shown below are obtained.

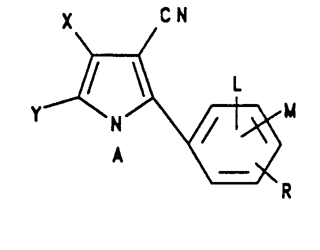

| **A** | **L** | **M** | **R** | **X** | **Y** | **mp°C** |
|---|---|---|---|---|---|---|
| CH₂COOC₂H₅ | H | 3-Cl | 4-Cl | Cl | Cl | 118-120 |
| CH₂COOC₆H₅ | H | 3-CL | 4-Cl | Cl | CF₃ | |
| CH₂COOC₆H₅-p-Cl | H | H | 4-CF₃ | Br | CF₃ | |
| CH₂OCOC(CH₃)₃ | H | 3-Cl | 4-Cl | Cl | Cl | |
| CH₂OCOCH₃ | H | H | 4-CF₃ | Cl | Cl | |
| CH₂CN | H | H | 4-Cl | Br | CF₃ | 142-144 |
| CH₂CN | H | H | 4-CF₃ | Cl | Cl | 137-139 |
| CH₂CN | H | H | 4-Br | Br | CF₃ | 110-113 |

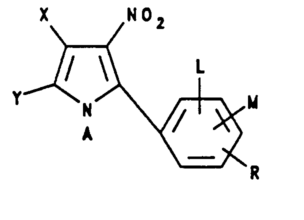

| **A** | **L** | **M** | **R** | **X** | **Y** | **mp°C** |
|---|---|---|---|---|---|---|
| CH₂OCOC(CH)₃ | H | 3-Cl | 4-Cl | H | CF₃ | 116-119 |
| CH₂OCOC₆H₅ | H | H | 4-Cl | Cl | | |

### EXAMPLE 2

### Insecticide and acaricide evaluations

All tests are performed using technical materials. All concentrations reported herein are in terms of active ingredient. All tests are kept at 27°C.

### Spodoptera eridania, 3rd instar larvae, souther armyworm

A Sieval lima bean leaf expanded to 7-8 cm in length is dipped in the test suspension with agitation for 3 seconds and placed in a hood to dry. The leaf is then placed in a 100x10 mm petri dish containing a damp filter paper on the bottom and ten 3rd instar caterpillars. The dish is maintained for 5 days before observations are made of mortality, reduced feeding, or any interference with normal moulting.

### Spodoptera eridania, 7-day residual

The plants treated in the above Test are maintained under high intensity lamps in the greenhouse for 7 days. These lamps duplicate the effects of a bright sunny day in June in New Jersey and are kept on for 14 hour day length. After 7 days, the foliage is sampled and assayed as in the above-said test.

### Aphis fabae, mixed instar, bean aphid

Pots containing single nasturtium plants (Tropaeolum sp.) about 5 cm tall are infested with about 100-200 aphids one day before the test. Each pot is sprayed with the test formulation for 2 revolutions of a 4 rpm turntable in a hood, using a #154 Devilbiss atomizer. The spray tip is held about 15 cm from the plant and the spray directed so as to give complete coverage of the plants and the aphids. The sprayed pots are set on their sides on white enamel trays and held for 2 days, following which mortality estimates are made.

### Tetranychus urticae(P-resistant strain),2-spotted spider mite

Sieva lima bean plants with primary leaves expaned to 7-8 cm are selected and cut back to one plant per pot. A small piece is cut from a leaf taken from the main colony and placed on each leaf of the test plants. This is done about 2 hours before treatment to allow the mites to move over to the test plant and to lay eggs. The size of the cut piece is varied to obtain about 100 mites per leaf. At the time of the treatment, the piece of leaf used to transfer the mites is removed and discarded. The mite-infested plants are dipped in the test formulation for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

### Diabrotic undecimpunctata howardi, 3rd instar southern corn rootworm

One cc of fine talc is placed in a 30 ml wide-mouth screw-top glass jar. One ml of the appropriate acetone suspension is pepetted onto the talc so as to provide 1.25 and 0.25 mg of active ingredient per jar. The jars are set under a gentle air flow until the acetone is evaporated. The dried talc is loosened, 1 cc of millet seed is added to serve as food for the insects and 25 ml of moist soil is added to each jar. The jar is capped and the contents thoroughly mixed on a Vortex Mixer. Following this, ten 3rd instar rootorms are added to each jar and the jars are loosely capped to allow air exchange for the larvae. The treatments are held for 6 days before mortality counts are made. Missing larvae are presumed dead, since they decompose rapidly and can not be found. The concentrations used in this test correspond approximately to 50 and 10 kg/ha, respectively.

### Rating Scale:

0 = no effect
1 = 10-25% kill
2 = 26-35% kill
3 = 36-45% kill
4 = 46-55% kill
5 = 56-65% kill
6 = 66-75% kill
7 = 76-85% kill
8 = 86-99% kill
9 = 100% kill
R - reduced feeding
The data obtained for the above-described evaluations are reported in Table I.

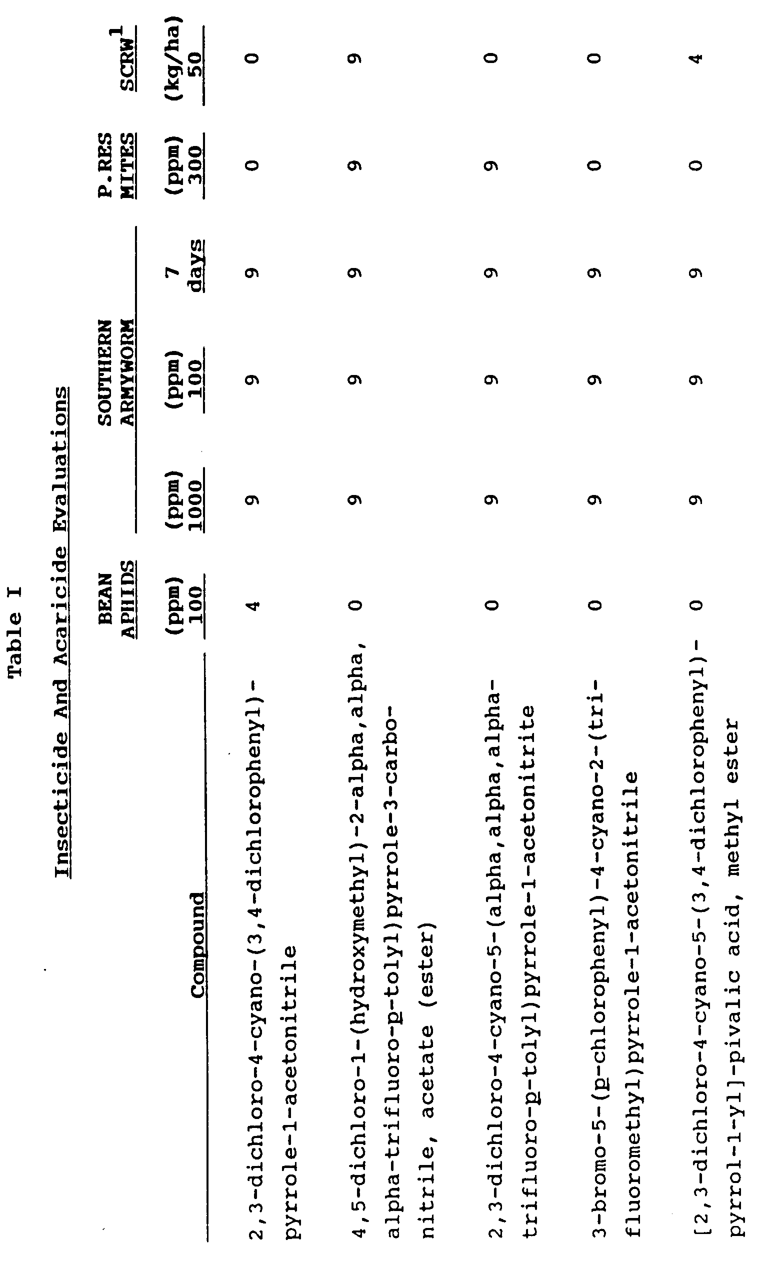

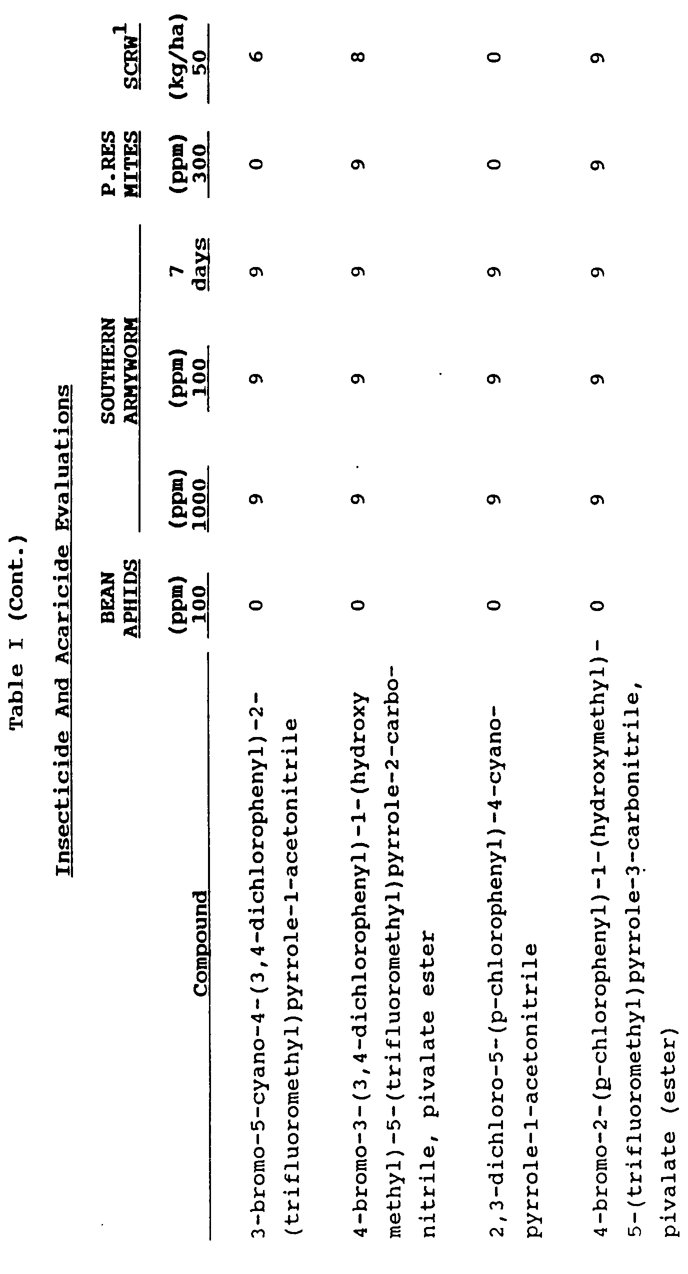

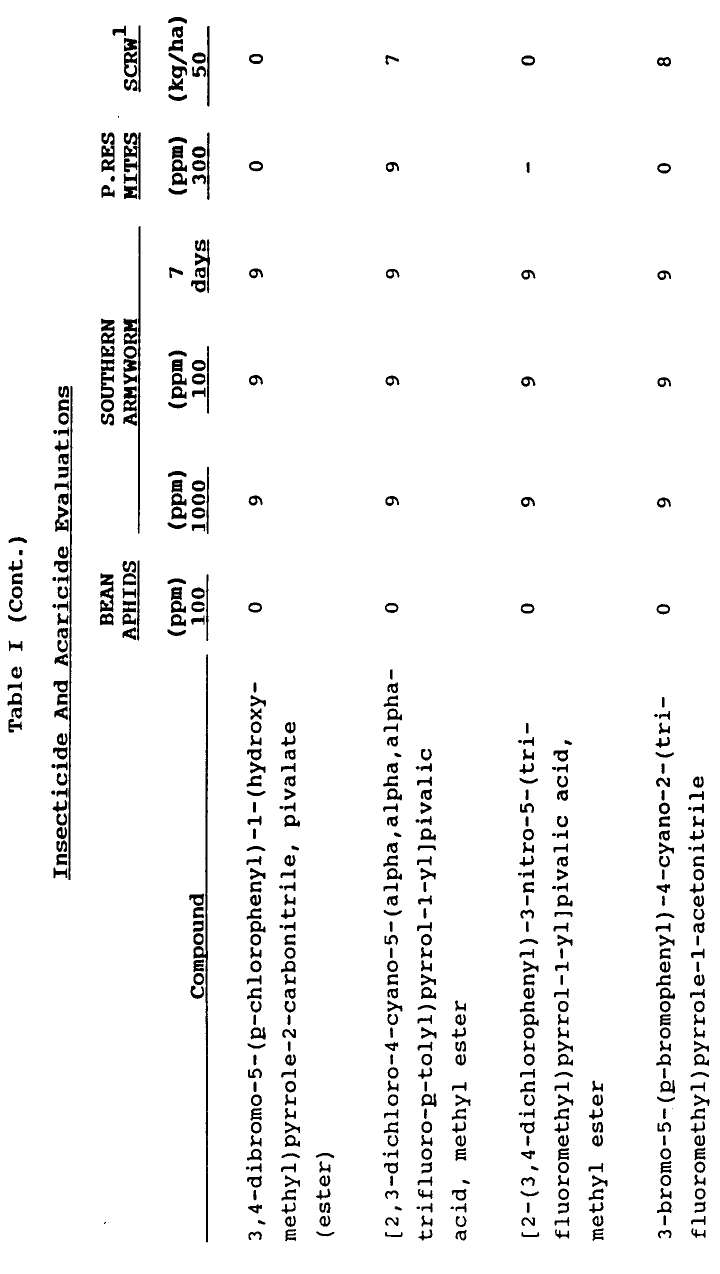

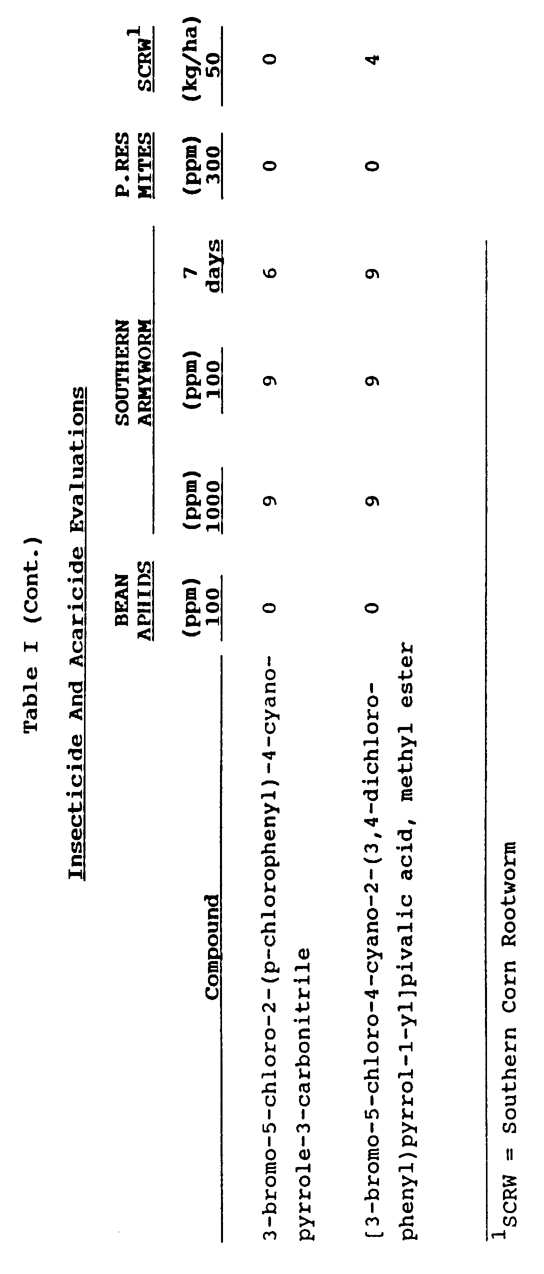

### EXAMPLE 3

### Insecticide evaluations

### Heliothis virescens, 3rd instar tobacco budworm

Cotton cotyledons are dipped in the test formulation and allowed to dry in a hood. When dry, each is cut into quarters and ten sections placed individually in 30 ml plastic medicine cups containing a 5-7 mm long piece of damp dental wick. One third-instar caterpillar is added to each cup and a cardboard lid placed on the cup. Treatments are maintained for 3 days before mortality counts and estimates of reduction in feeding damage are made.

### Empoasca abrupta, adults, western potato leafhopper

A Sieva lima bean leaf about 5 cm long is dipped in the test formulation for 3 seconds with agitation and placed in a hood to dry. The leaf is placed in a 100x10 mm petri dish containing a moist filter paper on the bottom. About 10 adult leafhoppers are added to each dish and the treatments are kept for 3 days before mortality counts are made.

### Blattella germanica, bait test, adult male German cockroach

A 0.1% bait is prepared by pipetting 1 ml of a 1000 ppm solution of the test compound in acetone onto 1 gram of cornmeal in a 30 ml wide-mouth bottle. The bait is dried by passing a gentle stream of air into the bottle. The bait is placed in a 1 pint wide-mouth Mason jar and ten adult male cockroaches are added. A screen lid is placed on the jar and a small piece of cotton soaked in 10% honey is put on the top of the screen lid. Mortality counts are made after 3 days.

### Blattela germanica, residue test, adult male German cockroach

One ml of a 1000 ppm acetone solution of the test material is pipetted slowly over the bottom of a 150 x 15 mm petri dish so as to give as uniform coverage as possible. After the deposit has dried, 10 adult male cockroaches are placed in each dish and the lid is added. Mortality counts are made after 3 days.

### Spodoptera eridania, systemic uptake, 3rd instar larvae, southern armyworm

The compound is formulated as an emulsion containing 0.1 gm of the test material, 0.2 gm of Emulphor EL-620® emulsifier, 10 ml of acetone and 90 ml of water. This is diluted 10-fold with water to give a 100 ppm emulsion for the test. Subsequent 10-fold dilutions are made with water as needed. Sieva lima bean plants, with the primary leaves expanded to a length of 7-8 cm, are cut off at least 3 cm above the soil level to avoid contamination with soil bacteria that will cause decay of the stem during the test. The cut stems are placed in the test emulsions and each stem is wrapped with a bit of cotton to hold the stem off the bottom of the bottle and to limit evaporation and volatilization of the compound. The test is maintained for 3 days at 27°C to allow the compounds to be taken up into the plant. Following this, one leaf is removed from the plant and placed in a 100 x 10 mm petri dish with 10 southern armyworms. Mortality counts and observations of feeding damage are made 3 and 5 days later.

### Empoasca abrupta, Adults, Western Potato Leafhoppers, Systemic Uptake

The compound is formulated as an emulsion containing 0.1 gm of the test material, 0.2 gm of Emulphor EL-620® emulsifier, 10 ml of acetone and 90 ml of water. This is diluted 10-fold with water to give a 100 ppm emulsion for the test. Subsequent 10-fold dilutions are made with water as needed. Sieva lima bean plants, with the primary leaves expanded to a length of 7-8 cm, are cut off at least 3 cm above the soil level to avoid contamination with soil bacteria that will cause decay of the stem during the test. The cut stems are placed in the test emulsions and each stem is wrapped with a bit of cotton to hold the stem off the bottom of the bottle and to limit evaporation and volatilization of the compound. The test is maintained for 3 days at 27°C to allow the compounds to be taken up into the plant. Following this, one leaf is removed from the plant and placed in a 100 x 10 mm petri dish with 10 adult Western Potato leafhoppers. After 3 days, mortality counts are made.

The rating scale for the above evaluations is the same as described in Example 2.

The data obtained are reported in Table II.

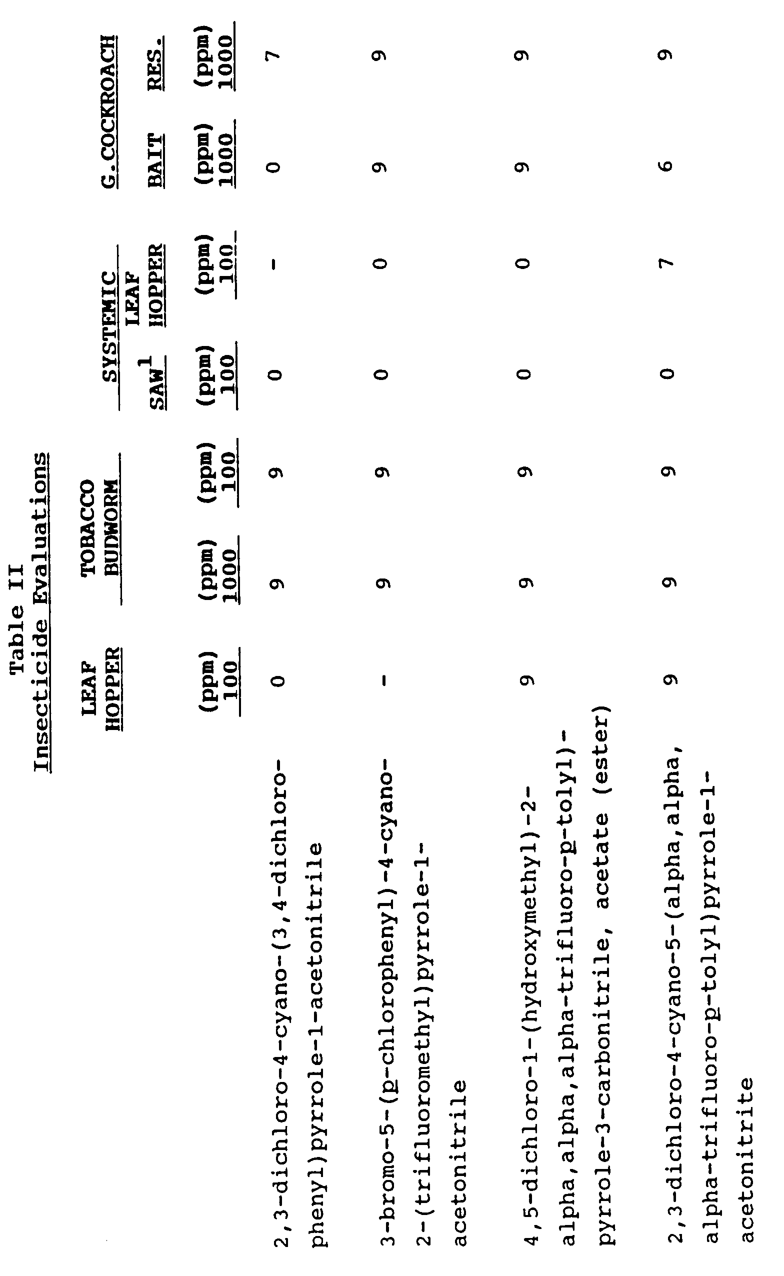

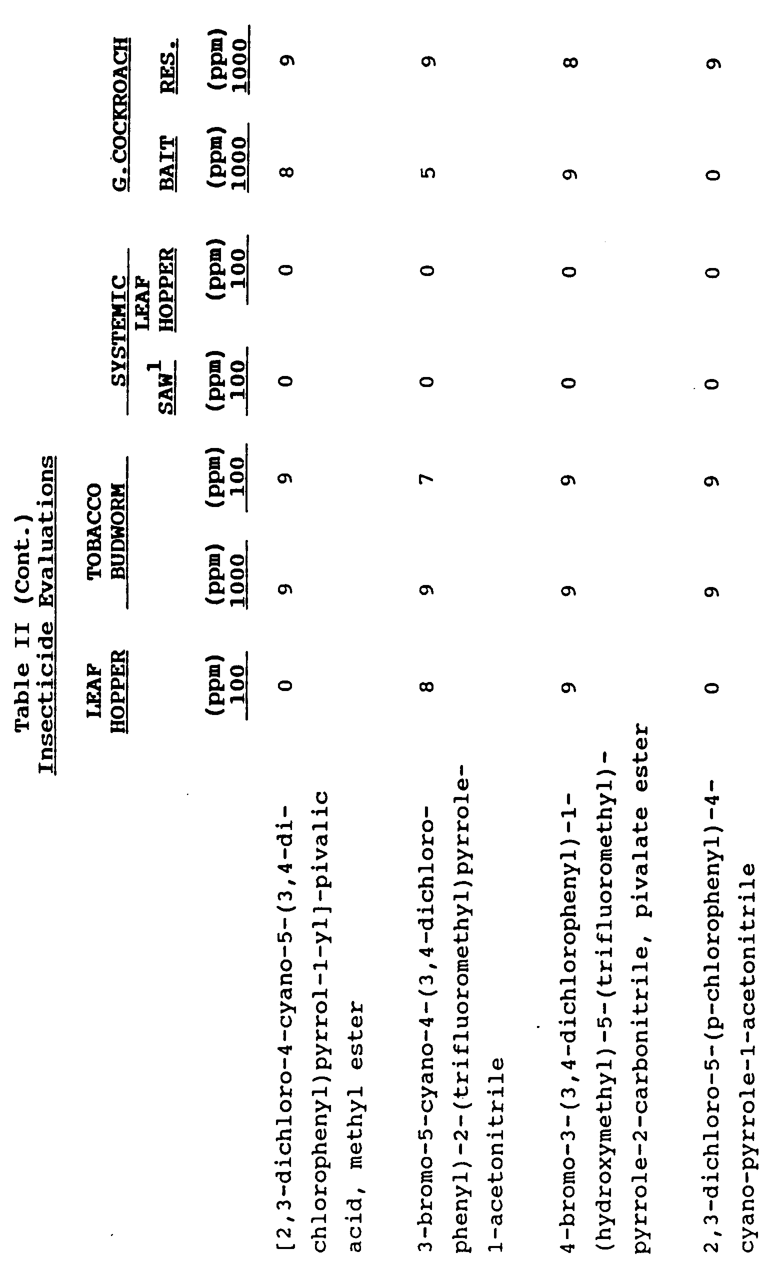

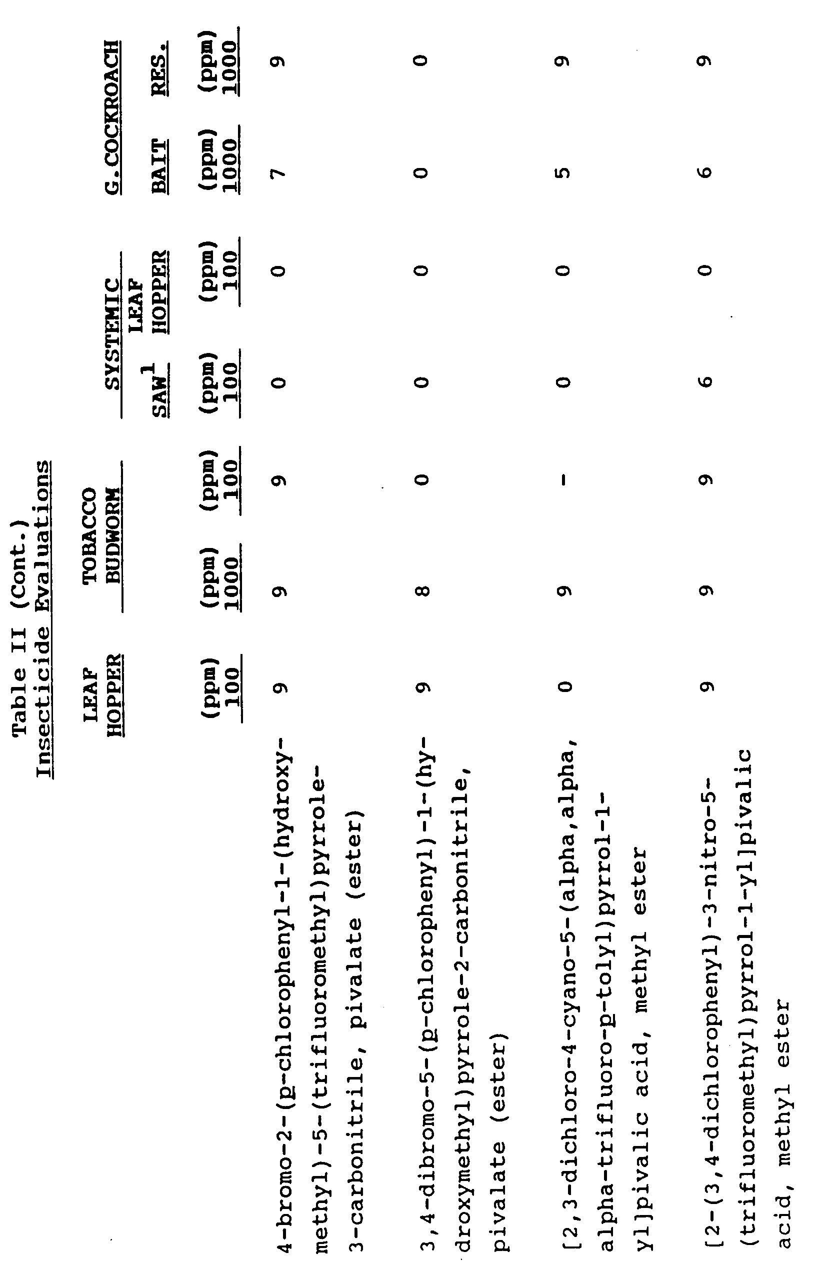

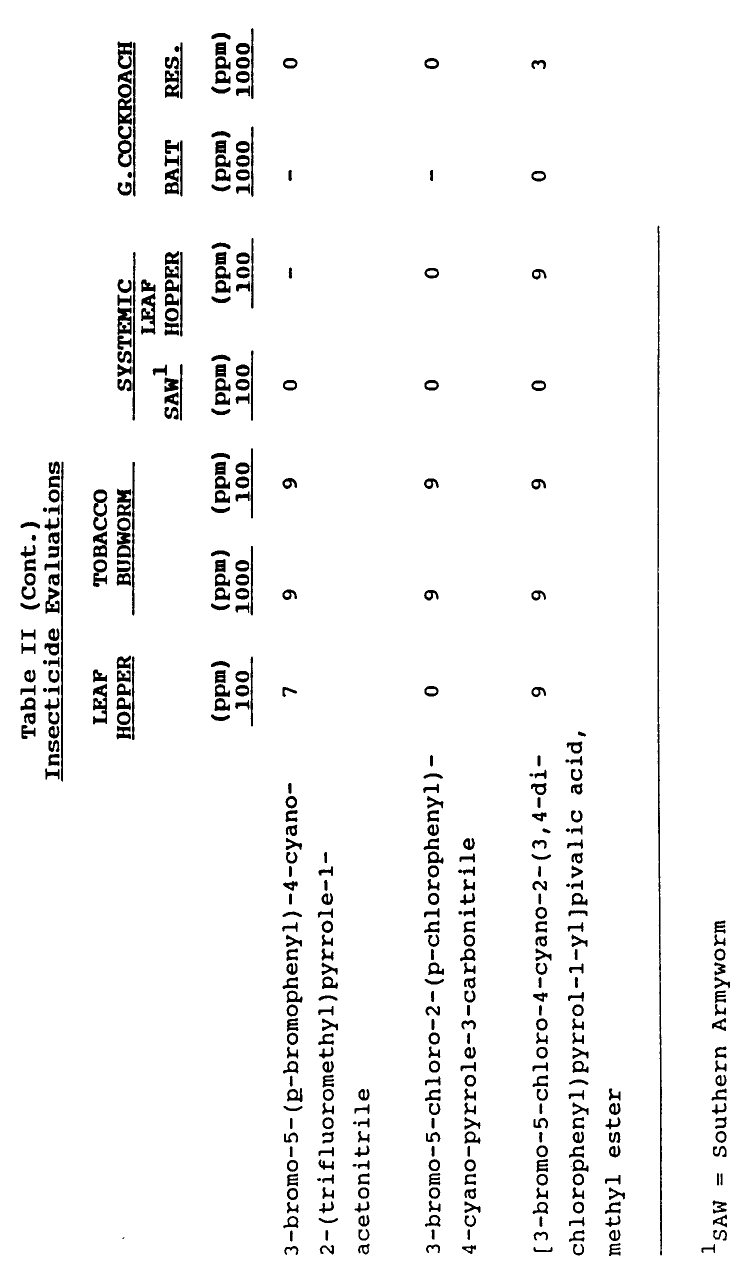

### EXAMPLE 4

### Evaluation of test compounds as nematicidal agents

Culture Maintenance: Cultures of C. elegans (Bristol strain from J. Lewis) are maintained on E. coli lawns on NG Agar Plates at 20°C. New cultures are established weekly.

Nematodes for testing are washed from 4-5 day old cultures using Fresh Ascaris Ringers Solution (FARS). The worms are further washed with FARS, containing gentamycin, to reduce bacterial contamination and centrifuged to separate worms from wash solution. This procedure is repeated three times. The washed worms are then added to C. briggsae Maintenance Medium (CbMM), from GIBCOa to which is added gentamycin (600 units/ml) and mycostatin (0.5 mg/ml).

The tests are then made with mixtures of three compounds, piggy-backed from another high capacity screening program to reduce additional labor and compound expenditures.

Compounds are dissolved in acetone and made up to volume with equal parts of water. The final test concentration of each compound in the mixture is 150 ppm. The test material is micropipetted (25 ul) into a single well of a 96-well sterile tissue culture plate (COSTAR)^{b} and the solvent allowed to evaporate. These "treated" plates are used immediately or stored in a freezer without apparent adverse effects on the compounds.

A freshly prepared volume (50 ug) of C. elegans in CbMM is micropipetted into each treated well and several control wells per plate. Culture plate are incubated at 20°C.

Observations for efficacy are made under a dissecting microscope at 4, 24 and 48 hours post-immersion. Immediately prior to reading the plate, it is gently tapped to stimulate the movement of the worms. Activity is judged subjectively, but semi-quantitatively, based on the drug effects on motility of the adults and larvae. The criteria are as follows: 8 = no motility, 7 = markedly reduced motility in approximately 95% of worms, 6 = reduced motility, 5 = slightly reduced motility, 0 = normal motility, same as controls. Other factors indicating activity are easily noted such as death, rigor mortis, contraction, coiling, paralysis, abnormal twitching, reduced worm population in 48 hours and other deviation from normal behavior.

### PROCEDURE FOR CAENORHABDITIS ELEGANS ASSAY

- Day 0: .Inoculate E. Coli-NG Agar Dish With 30-50 C. Elegans
.Incubate At 20°C.
- Day 4: .Harvest New C. Elegans Population
.Wash With Antibiotics
.Transfer To CbMM
.Add C. Elegans (25-100 UL) To "Medicated" Wells^{a}
.Observe For Activity At 4 Hours Post-Immersion
- Day 5: .Observe For Activity
- Day 6: .Observe For Activity

^{a} Medicated Wells May Be Prepared Fresh Or Earlier And Stores In Freezer

Data obtained in these tests are reported in Table III below.

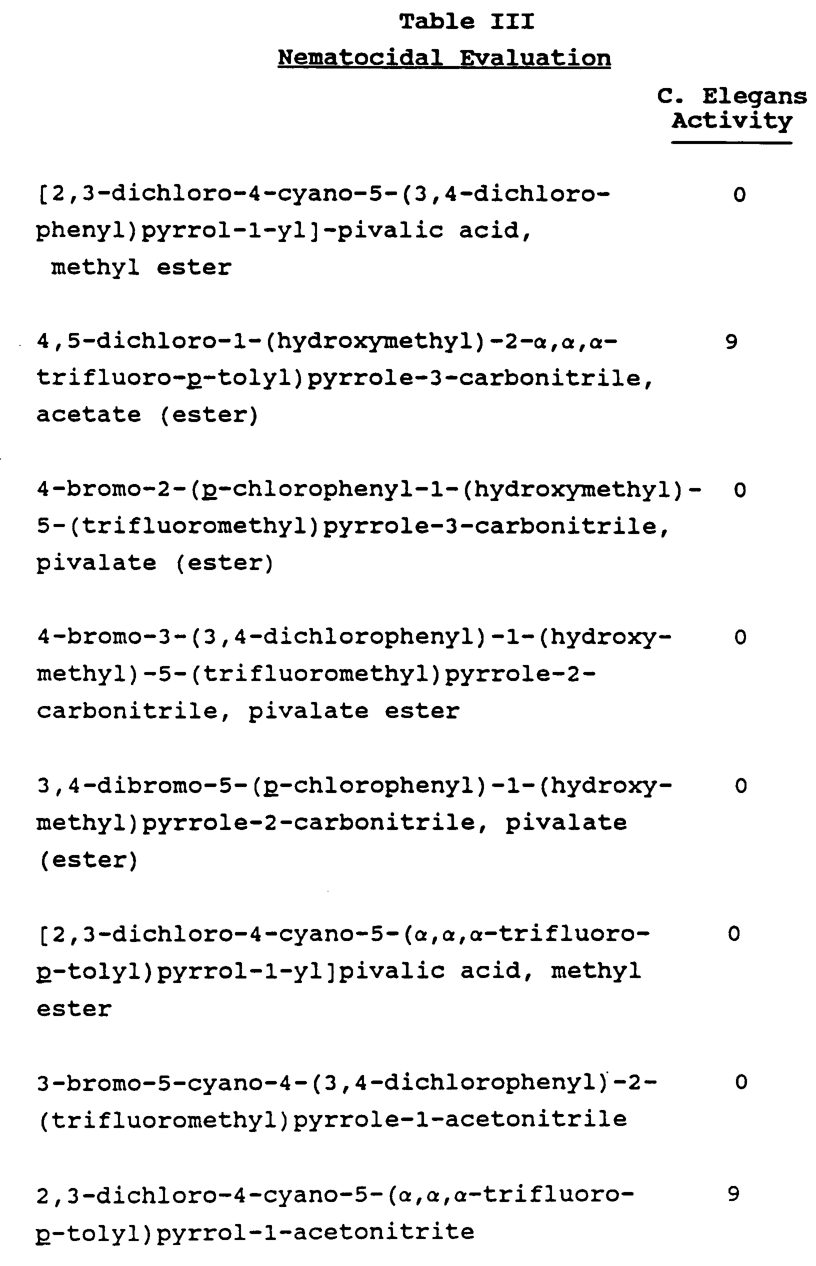

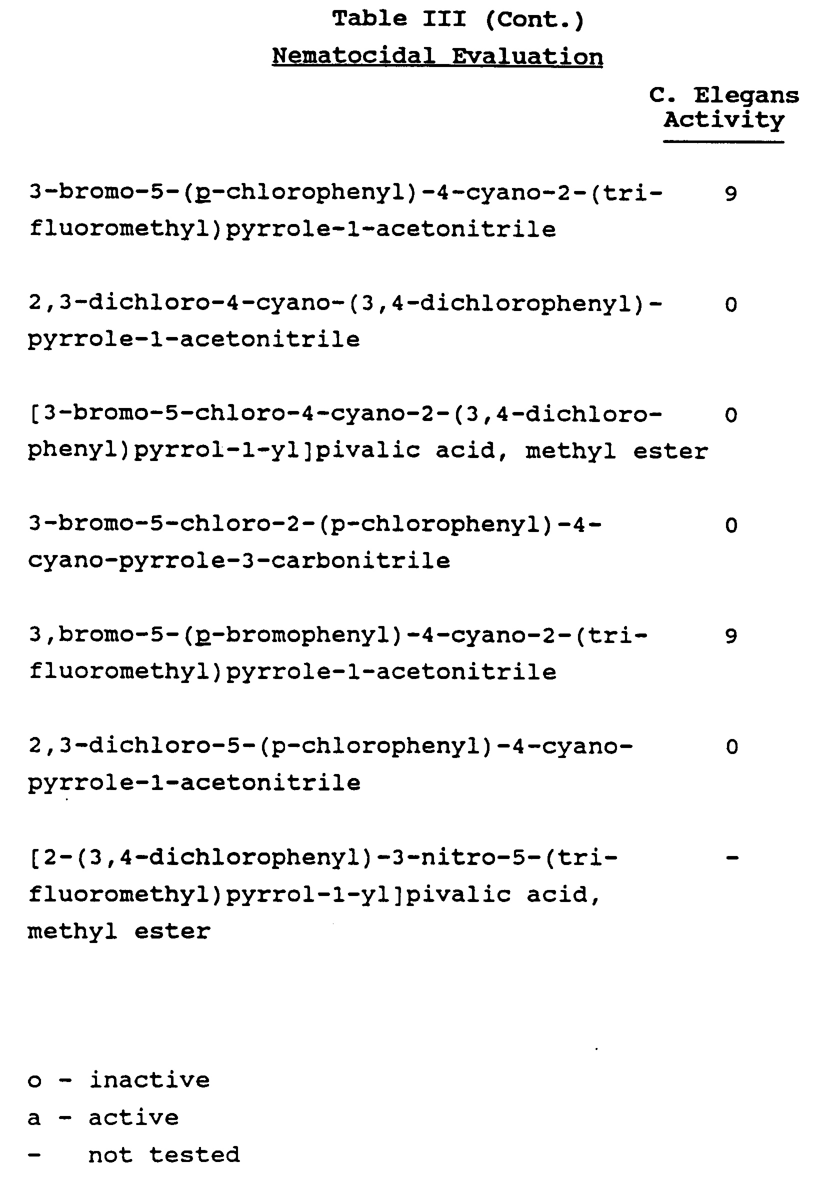

## Claims (Claims for the following Contracting State(s): AT, BE, CH, DE, DK, FR GB, GR, IT, LI, LU, NL, SE)

1. A compound having the formula I structure: 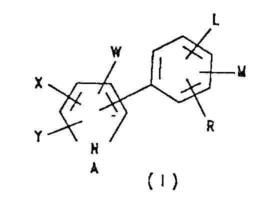 wherein
X is H, F, Cl, Br, I or CF₃;
Y is H, F, Cl, Br, I, CF₃ or CN;
W is CN or NO₂;
A is C₁-C₄ alkyl substituted with four halogen atoms, one cyano, one C₁-C₄ alkoxy group substituted with one to three halogen atoms or two C₁-C₄ alkoxy groups optionally substituted with one to three halogen atoms, one C₁-C₄ carbalkoxy, one C₁-C₆ alkylcarbonyloxy, one C₂-C₆ alkenylcarbonyloxy, one benzenecarbonyloxy or chloro, dichloro or methylsubstitutedbenzenecarbonyloxy;
L is H, F, Cl or Br and
M and R are each independently H, C₁-C₃ alkyl, C₁-C₃ alkoxy, C₁-C₃ alkylthio, C₁-C₃ alkylsulfinyl, C₁-C₃ alkylsulfonyl, cyano, F, Cl, Br, I, nitro, CF₃, R₁CF₂Z, R₂CO or NR₃R₄ and when M and R are on adjacent positions and taken with the carbon atoms to which they are attached they may form a ring in which MR represents the structure:
-OCH₂O-, -OCF₂O- or 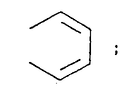
Z is S(O)n or O;
R₁ is H, F, CHF₂, CHFC1, or CF₃;
R₂ is C₁-C₃ alkyl, C₁-C₃ alkoxy or NR₃R₄;
R₃ is H or C₁-C₃ alkyl;
R₄ is H, C₁-C₃ alkyl or R₅CO;
R₅ is H or C₁-C₃ alkyl and
n is an integer of 0, 1 or 2;
provided that when X and Y are H and A is C₁-C₄ alkyl substituted with one cyano or one C₁₋₄ carbalkoxy, then the 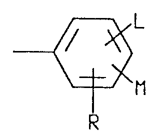 substituent must be fixed to one of the two positions on the pyrrole ring adjacent to the nitrogen atom.

2. The compound according to claim 1 wherein
W is CN or NO₂;
X and Y are each Cl, CF₃ or Br;
R is F, Cl, Br, CF₃ or OCF₃;
M is H, F, Cl or Br and
L is H or F.

3. The compound according to claim 1 wherein said compound has the structure: 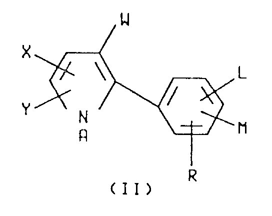 and A, L, M, R, W, X and Y are as described in claim 1.

4. The compound according to claim 1 wherein said compound has the structure: 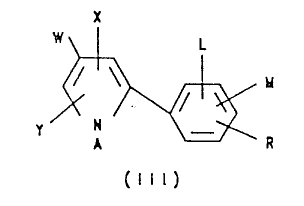 and A, L, M, R, W, X and Y are as described in claim 1.

5. A method for controlling insects, nematodes and acarina comprising contacting said insects, nematodes and acarina, their breeding grounds, food supply or habitat with an insecticidally, nematicidally or acaricidally effective amount of a compound of formula I wherein X, Y, W, A, L, M and R are as described in Claim 1.

6. The method according to Claim 5 where in the compound of formula I has the structure as described in Claim 2.

7. A method for protecting growing plants from attack by insects, nematodes and acarina, comprising applying to the foliage of said plants or to the soil or water in which they are growing, an insecticidally, nematicidally or acaricidally, effective amount of a compound of formula I wherein X, Y, W, A, L, M and R are as described in Claim 1.

8. The method according to claim 7 wherein said compound is applied to said plants or the soil in which they are growing, at a rate of about 0.125 kg/ha to about 4.0 kg/ha.

9. The method according to claim 7 wherein said formula I compound is applied to the foliage of said plants or the soil or water in which they are growing, in the form of a liquid composition containing from about 10 ppm to about 10,000 ppm of said formula I compound.

## Claims (Claims for the following Contracting State(s): ES)

1. A process for the preparation of a compound of formula I 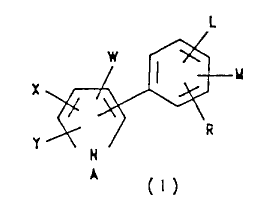 wherein
X is H, F, Cl, Br, I or CF₃;
Y is H, F, Cl, Br, I, CF₃ or CN;
W is CN or NO₂;
A is C₁-C₄ alkyl substituted with four halogen atoms, one cyano, one C₁-C₄ alkoxy group substituted with one to three halogen atoms or two C₁-C₄ alkoxy groups optionally substituted with one to three halogen atoms, one C₁-C₄ carbalkoxy, one C₁-C₆ alkylcarbonyloxy, one C₂-C₆ alkenylcarbonyloxy, one benzenecarbonyloxy or chloro, dichloro or methylsubstitutedbenzenecarbonyloxy;
L is H, F, Cl or Br and
M and R are each independently H, C₁-C₃ alkyl, C₁-C₃ alkoxy, C₁-C₃ alkylthio, C₁-C₃ alkylsulfinyl, C₁-C₃ alkylsulfonyl, cyano, F, Cl, Br, I, nitro, CF₃, R₁CF₂Z, R₂CO or NR₃R₄ and when M and R are on adjacent positions and taken with the carbon atoms to which they are attached they may form a ring in which MR represents the structure:
-OCH₂O-, -OCF₂O- or 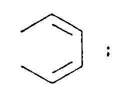
Z is S(O)n or O;
R₁ is H, F, CHF₂, CHFC1, or CF₃;
R₂ is C₁-C₃ alkyl, C₁-C₃ alkoxy or NR₃R₄;
R₃ is H or C₁-C₃ alkyl;
R₄ is H, C₁-C₃ alkyl or R₅CO;
R₅ is H or C₁-C₃ alkyl and
n is an integer of 0, 1 or 2;
provided that when X and Y are H and A is C₁-C₄ alkyl substituted with one cyano or one C₁₋₄ carbalkoxy, then the 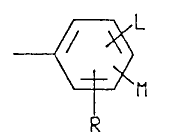 substituent must be fixed to one of the two positions on the pyrrole ring adjacent to the nitrogen atom, which comprises reacting a compound of formula VIII 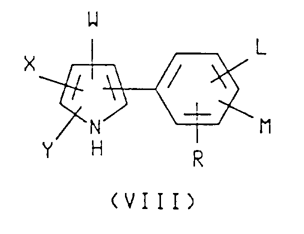 with an appropriate alkylating agent and a suitable base to afford the desired formula I compound.

2. The process according to claim 1 wherein
W is CN or NO₂;
X and Y are each Cl, CF₃ or Br;
R is F, Cl, Br, CF₃ or OCF₃;
M is H, F, Cl or Br and
L is H or F.

3. The process according to claim 1 wherein said compound has the structure: 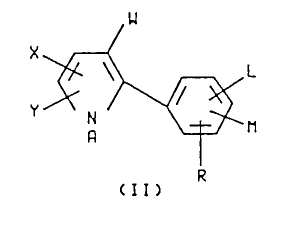 and A, L, M, R, W, X and Y are as described in claim 1.

4. The process according to claim 1 wherein said compound has the structure: 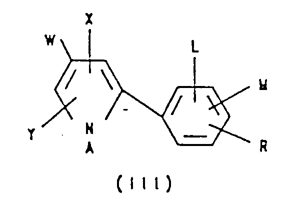 and A, L, M, R, W, X and Y are as described in claim 1.

5. A method for controlling insects, nematodes and acarina comprising contacting said insects, nematodes and acarina, their breeding grounds, food supply or habitat with an insecticidally, nematicidally or acaricidally effective amount of a compound of formula I wherein X, Y, W, A, L, M and R are as described in Claim 1.

6. The method according to Claim 5 where in the compound of formula I has the structure as described in Claim 2.

7. A method for protecting growing plants from attack by insects, nematodes and acarina, comprising applying to the foliage of said plants or to the soil or water in which they are growing, an insecticidally, nematicidally or acaricidally, effective amount of a compound of formula I wherein X, Y, W, A, L, M and R are as described in Claim 1.

8. The method according to claim 7 wherein said compound is applied to said plants or the soil in which they are growing, at a rate of about 0.125 kg/ha to about 4.0 kg/ha.

9. The method according to claim 7 wherein said formula I compound is applied to the foliage of said plants or the soil or water in which they are growing, in the form of a liquid composition containing from about 10 ppm to about 10,000 ppm of said formula I compound.

## Patentansprüche (Patentansprüche für folgende(n) Vertragsstaat(en): AT, BE, CH, DE, DK, FR, GB, GR, IT, LI, LU, NL, SE)

1. Verbindung der Formel I: 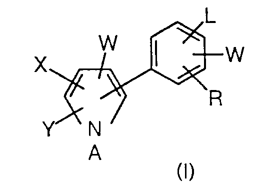 worin X H, F, Cl, Br, I oder CF₃ ist;
Y H, F, Cl, Br, I, CF₃ oder CN ist;
W CN oder NO₂ ist;
A C₁-C₄-Alkyl, substituiert mit vier Halogenatomen, eine Cyangruppe, eine C₁-C₄-Alkoxygruppe, substituiert mit ein bis drei Halogenatomen oder zwei C₁-C₄-Alkoxygruppen, gegebenenfalls substituiert mit ein bis drei Halogenatomen, eine C₁-C₄-Carbalkoxy, ein C₁-C₆-Alkylcarbonyloxy, ein C₂-C₆-Alkenylcarbonyloxy, ein Benzolcarbonyloxy oder chlor-, dichlor- oder methyl-substituiertes Benzolcarbonyloxy ist;
L H, F, Cl oder Br ist und
M und R jeweils unabhängig H, C₁-C₃-Alkyl, C₁-C₃-Alkoxy, C₁-C₃-Alkylthio, C₁-C₃-Alkylsulfinyl, C₁-C₃-Alkylsulfonyl, Cyan, F, Cl, Br, I, Nitro, CF₃, R₁CF₂Z, R₂CO oder NR₃NR₄ sind und, wenn M und R sich an benachbarten Positionen befinden, sie zusammen mit den Kohlenstoffatomen, an die sie gebunden sind, einen Ring bilden können, in dem MR die Struktur repräsentiert:
-OCH₂O-, -OCF₂O- oder 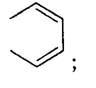 Z S(O)ₙ oder O ist;
R₁ H, F, CHF₂, CHFCl oder CF₃ ist;
R₂ C₁-C₃-Alkyl, C₁-C₃-Alkoxy oder NR₃R₄ ist;
R₃ H oder C₁-C₃-Alkyl ist;
R₄ H, C₁-C₃-Alkyl oder R₅CO ist;
R₅ H oder C₁-C₃-Alkyl ist, und
n eine ganze Zanl von 0, 1 oder 2 ist,
unter der Bedingung, daß, wenn X und Y H sind und A ein C₁-C₄-Alkyl, substituiert mit einem Cyan, oder ein C₁-C₄-Carbalkoxy ist, der Substituent an eine der beiden Positionen auf dem 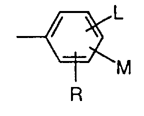 Pyrrolring benachbart dem Stickstoffatom gebunden sein muß.

2. Verbindung nach Anspruch 1, worin
W CN oder NO₂ ist;
X und Y jeweils Cl, CF₃ oder Br sind;
R F, Cl, Br, CF₃ oder OCF₃ ist;
M H, F, Cl oder Br ist, und
L H oder F ist.

3. Verbindung nach Anspruch 1, die die Struktur hat: 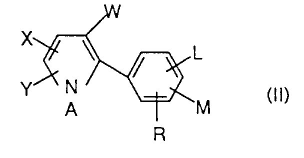 worin A, L, M, R, W, X und Y die in Anspruch 1 angegebene Bedeutung haben.

4. Verbindung nach Anspruch 1, die die Struktur hat: 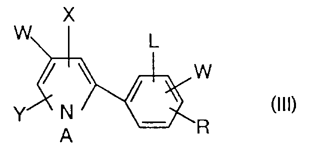 worin A, L, M, R, W, X und Y die in Anspruch 1 angegebene Bedeutung haben.

5. Verfahren zum Bekämpfen von Insekten, Nematoden und Milben, umfassend das Inberührungbringen der Insekten, Nematoden und Milben, ihrer Brutgebiete, ihres Futters oder Lebensraumes mit einer insektizid, nematizid oder akarizid wirksamen Menge einer Verbindung der Formel I, worin X, Y, W, A, L, M und R die in Anspruch 1 angegebene Bedeutung haben.

6. Verfahren nach Anspruch 5, worin die Verbindung der Formel I die Struktur von Anspruch 2 hat.

7. Verfahren zum Schützen wachsender Pflanzen vor Angriffen durch Insekten, Nematoden und Milben, umfassend das Aufbringen einer insektizid, nematizid oder akarizid wirksamen Menge einer Verbindung der Formel I, worin X, Y, W, A, L, M und R die in Anspruch 1 angegebene Bedeutung haben, auf das Laub der Pflanzen oder den Boden oder das Wasser, in dem sie wachsen.

8. Verfahren nach Anspruch 7, worin die Verbindung in einer Rate von etwa 0,125 kg/ha bis etwa 4,0 kg/ha auf die Pflanzen oder den Boden, in dem sie wachsen, angewendet wird.

9. Verfahren nach Anspruch 7, worin die Verbindung der Formel I in Form einer flüssigen Zusammensetzung, enthaltend von etwa 10 ppm bis etwa 1.000 ppm der Verbindung der Formel I, auf das Laub der Pflanzen oder den Boden oder das Wasser, in dem sie wachsen, angewendet wird.

## Patentansprüche (Patentansprüche für folgende(n) Vertragsstaat(en): ES)

1. Verfahren zum Herstellen einer Verbindung der Formel I: 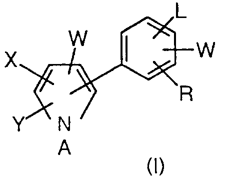 worin X H, F, Cl, Br, I oder CF₃ ist;
Y H, F, Cl, Br, I, CF₃ oder CN ist;
W CN oder NO₂ ist;
A C₁-C₄-Alkyl, substituiert mit vier Halogenatomen, eine Cyangruppe, eine C₁-C₄-Alkoxygruppe, substituiert mit ein bis drei Halogenatomen oder zwei C₁-C₄-Alkoxygruppen, gegebenenfalls substituiert mit ein bis drei Halogenatomen, eine C₁-C₄-Carbalkoxy, ein C₁-C₆-Alkylcarbonyloxy, ein C₂-C₆-Alkenylcarbonyloxy, ein Benzolcarbonyloxy oder chlor-, dichlor- oder methyl-substituiertes Benzolcarbonyloxy ist;
L H, F, Cl oder Br ist, und
M und R jeweils unabhängig H, C₁-C₃-Alkyl, C₁-C₃-Alkoxy, C₁-C₃-Alkylthio, C₁-C₃-Alkylsulfinyl, C₁-C₃-Alkylsulfonyl, Cyan, F, Cl, Br, I, Nitro, CF₃, R₁CF₂Z, R₂CO oder NR₃NR₄ sind und, wenn M und R sich an benachbarten Positionen befinden, sie zusammen mit den Kohlenstoffatomen, an die sie gebunden sind, einen Ring bilden können, in dem MR die Struktur repräsentiert:
-OCH₂O-, -OCF₂O- oder 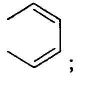 Z S(O)ₙ oder O ist;
R₁ H, F, CHF₂, CHFCl oder CF₃ ist;
R₂ C₁-C₃-Alkyl, C₁-C₃-Alkoxy oder NR₃R₄ ist;
R₃ H oder C₁-C₃-Alkyl ist;
R₄ H, C₁-C₃-Alkyl oder R₅CO ist;
R₅ H oder C₁-C₃-Alkyl ist, und
n eine ganze Zahl von 0, 1 oder 2 ist,
unter der Bedingung, daß, wenn X und Y H sind und A ein C₁-C₄-Alkyl, substituiert mit einem Cyan, oder ein C₁-C₄-Carbalkoxy ist, der substituent an eine der beiden Positionen auf dem 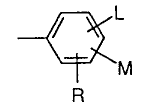 Prrolring benachbart dem Stickstoffatom gebunden sein muß, umfassend das Umsetzen einer Verbindung der Formel VIII 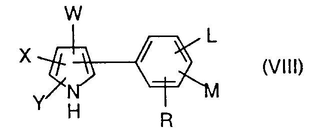 mit einem geeigneten Alkylierungsmittel und einer geeigneten Base, um die erwünschte Verbindung der Formel I zu bilden.

2. Verfahren nach Anspruch 1, worin
W CN oder NO₂ ist;
X und Y jeweils Cl, CF₃ oder Br sind;
R F, Cl, Br, CF₃ oder OCF₃ ist;
M H, F, Cl oder Br ist, und
L H oder F ist.

3. Verfahren nach Anspruch 1, worin die Verbindung die Struktur hat: 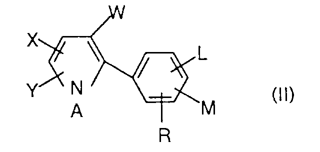 worin A, L, M, R, W, X und Y die in Anspruch 1 angegebene Bedeutung haben.

4. Verfahren nach Anspruch 1, worin die Verbindung die Struktur hat: 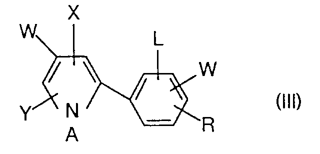 worin A, L, M, R, W, X und Y die in Anspruch 1 angegebene Bedeutung haben.

5. Verfahren zum Bekämpfen von Insekten, Nematoden und Milben, umfassend das Inberührungbringen der Insekten, Nematoden und Milben, ihrer Brutgebiete, ihres Futters oder Lebensraumes mit einer insektizid, nematizid oder akrizid wirksamen Menge einer Verbindung der Formel I, worin X, Y, W, A, L, M und R die in Anspruch 1 angegebene Bedeutung haben.

6. Verfahren nach Anspruch 5, worin die Verbindung der Formel I die Struktur von Anspruch 2 hat.

7. Verfahren zum Schützen wachsender Pflanzen vor Angriffen durch Insekten, Nematoden und Milben, umfassend das Aufbringen einer insektizid, nematizid oder akarizid wirksamen Menge einer Verbindung der Formel I, worin X, Y, W, A, L, M und R die in Anspruch 1 angegebene Bedeutung haben, auf das Laub der Pflanzen oder den Boden oder das Wasser, in dem sie wachsen.

8. Verfahren nach Anspruch 7, worin die Verbindung in einer Rate von etwa 0,125 kg/ha bis etwa 4,0 kg/ha auf die Pflanzen oder den Boden, in dem sie wachsen, angewendet wird.

9. Verfahren nach Anspruch 7, worin die Verbindung der Formel I in Form einer flüssigen Zusammensetzung, enthaltend von etwa 10 ppm bis etwa 1.000 ppm der Verbindung der Formel I, auf das Laub der Pflanzen oder den Boden oder das Wasser, in dem sie wachsen, angewendet wird.

## Revendications (Revendications pour l'(les) Etat(s) contractant(s) suivant(s): AT, BE, CH, DE, DK, FR, GB, GR, IT, LI, LU, NL, SE)

1. Un composé ayant la structure de formule I : 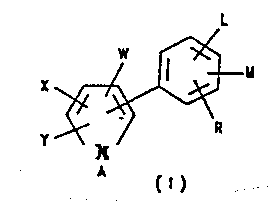 dans laquelle
X est H, F, Cl, Br, I ou CF₃ ;
Y est H, F, Cl, Br, I, CF₃ ou CN ;
W est CN ou NO₂ ;
A est un radical alkyle en C₁-C₄ substitué par: quatre atomes d'halogène, un radical cyano, un radical alcoxy en C₁-C₄ substitué par un à trois atomes d'halogène ou deux radicaux alcoxy en C₁-C₄ facultativement substitués par un à trois atomes d'halogène, un radical carbalcoxy en C₁-C₄, un radical alkylcarbonyloxy en C₁-C₆, un radical alcénylcarbonyloxy en C₂-C₆, un radical benzènecarbonyloxy ou benzènecarbonyloxy à substitution chloro, dichloro ou méthyle ;
L est H, F, Cl ou Br, et
M et R sont chacun indépendamment H, un radical alkyle en C₁-C₃, alcoxy en C₁-C₃, alkylthio en C₁-C₃, alkylsulfinyle en C₁-C₃, alkylsulfonyle en C₁-C₃, cyano, F, Cl, Br, I, nitro, CF₃, R₁CF₂Z, R₂CO ou NR₃R₄, et si M et R sont situés à des positions adjacentes et pris avec les atomes de carbone auxquels ils sont liés, ils peuvent former un cycle dans lequel MR représente la structure :
-OCH₂O-, -OCF₂O- ou 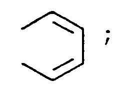
Z est S(O)ₙ ou O ;
R₁ est H, F, CHF₂, CHFCl ou CF₃ ;
R₂ est un radical alkyle en C₁-C₃, alcoxy en C₁-C₃ ou NR₃R₄ ;
R₃ est H ou un radical alkyle en C₁-C₃ ;
R₄ est H, un radical alkyle en C₁-C₃ ou R₅CO ;
R₅ est H ou un radical alkyle en C₁-C₃ ; et
n est un nombre entier égal à 0, 1 ou 2;
à condition que si X et Y sont H et A est un radical alkyle en C₁-C₄ substitué par un radical cyano ou un radical carbalcoxy en C₁-C₄, alors le substituant 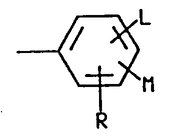 soit fixé à l'une des deux positions du noyau de pyrrole qui sont adjacentes à l'atome d'azote.

2. Le composé selon la revendication 1 dans lequel
W est CN ou NO₂ ;
X et Y sont chacun Cl, CF₃ ou Br ;
R est F, Cl, Br, CF₃ ou OCF₃ ;
M est H, F, Cl ou Br, et
L est H ou F.

3. Le composé selon la revendication 1, dans lequel ledit composé a la structure : 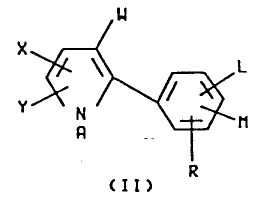 et A, L, M, R, W, X et Y sont tels que définis dans la revendication 1.

4. Le composé selon la revendication 1, dans lequel ledit composé a la structure : 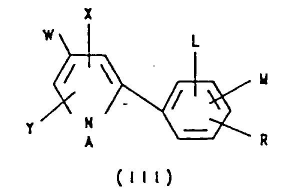 et A, L, M, R, W, X et Y sont tels que définis dans la revendication 1.

5. Un procédé pour combattre des insectes, nématodes et acariens, comprenant la mise en contact desdits insectes, nématodes et acariens, de leurs lieux de reproduction, sources de nourriture ou habitat avec une quantité à effet insecticide, nématicide ou acaricide d'un composé de formule I où X, Y, W, A, L, M et R sont tels que définis dans la revendication 1.

6. Le procédé selon la revendication 5, dans lequel le composé de formule I a la structure telle que décrite dans la revendication 2.

7. Un procédé pour protéger des plantes en croissance contre une attaque par des insectes, nématodes et acariens, comprenant l'application au feuillage desdites plantes, ou au sol ou a l'eau où elles poussent, d'une quantité à effet insecticide, nématicide ou acaricide d'un composé de formule I où X, Y, W, A, L, M et R sont tels que définis dans la revendication 1.

8. Le procédé selon la revendication 7, dans lequel ledit composé est applique auxdites plantes ou au sol dans lequel elles poussent, à une dose d'environ 0,125 kg/ha à environ 4,0 kg/ha.

9. Le procédé selon la revendication 7, dans lequel ledit composé de formule I est appliqué au feuillage desdites plantes ou au sol ou à l'eau où elles poussent sous la forme d'une composition liquide contenant environ 10 ppm à environ 10 000 ppm dudit composé de formule I.

## Revendications (Revendications pour l'(les) Etat(s) contractant(s) suivant(s): ES)

1. Procédé pour la préparation d'un composé de formule I 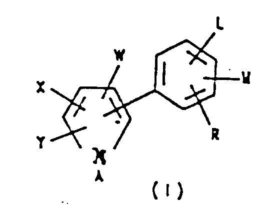 dans laquelle
X est H, F, Cl, Br, I ou CF₃ ;
Y est H, F, Cl, Br, I, CF₃ ou CN ;
W est CN ou NO₂ ;
A est un radical alkyle en C₁-C₄ substitué par: quatre atomes d'halogène, un radical cyano, un radical alcoxy en C₁-C₄ substitué par un à trois atomes d'halogène ou deux radicaux alcoxy en C₁-C₄ facultativement substitués par un à trois atomes d'halogène, un radical carbalcoxy en C₁-C₄, un radical alkylcarbonyloxy en C₁-C₆, un radical alcénylcarbonyloxy en C₂-C₆, un radical benzènecarbonyloxy ou benzènecarbonyloxy à substitution chloro, dichloro ou méthyle ;
L est H, F, Cl ou Br, et
M et R sont chacun indépendamment H, un radical alkyle en C₁-C₃, alcoxy en C₁-C₃, alkylthio en C₁-C₃, alkylsulfinyle en C₁-C₃, alkylsulfonyle en C₁-C₃, cyano, F, Cl, Br, I, nitro, CF₃, R₁CF₂Z, R₂CO ou NR₃R₄, et si M et R sont situés à des positions adjacentes et pris avec les atomes de carbone auxquels ils sont liés, ils peuvent former un cycle dans lequel MR représente la structure :
-OCH₂O-, -OCF₂O- ou 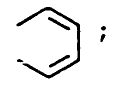
Z est S(O)ₙ ou O ;
R₁ est H, F, CHF₂, CHFCl ou CF₃ ;
R₂ est un radical alkyle en C₁-C₃, alcoxy en C₁-C₃ ou NR₃R₄ ;
R₃ est H ou un radical alkyle en C₁-C₃ ;
R₄ est H, un radical alkyle en C₁-C₃ ou R₅CO ;
R₅ est H ou un radical alkyle en C₁-C₃ ; et
n est un nombre entier égal à, O, 1 ou 2;
à condition que si X et Y sont H et A est un radical alkyle en C₁-C₄ substitué par un radical cyano ou un radical carbalcoxy en C₁-C₄, alors le substituant 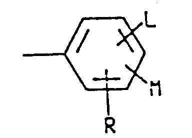 soit fixé à l'une des deux positions du noyau de pyrrole qui sont adjacentes à l'atome d'azote
comprenant la réaction d'un composé de formule VIII 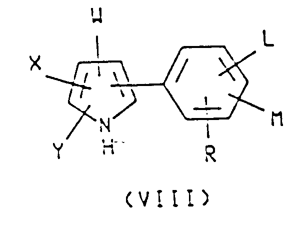 avec un agent alkylant approprié et une base adaptée pour conduire au composé souhaité de formule I.

2. Procédé selon la revendication 1 dans lequel
W est CN ou NO₂ ;
X et Y sont chacun Cl, CF₃ ou Br ;
R est F, Cl, Br, CF₃ ou OCF₃ ;
M est H, F, Cl ou Br, et
L est H ou F.

3. Procédé selon la revendication 1 dans lequel ledit composé a la structure : 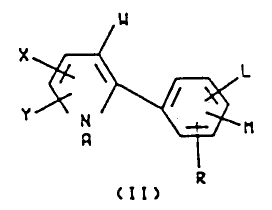 et A, L, M, R, W, X et Y sont tels que définis dans la revendication 1.

4. Procédé selon la revendication 1 dans lequel ledit composé a la structure : 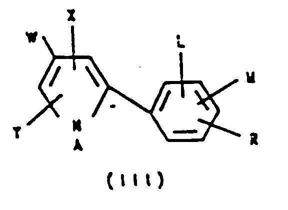 et A, L, M, R, W, X et Y sont tels que définis dans la revendication 1.

5. Un procédé pour combattre des insectes, nématodes et acariens, comprenant la mise en contact desdits insectes, nématodes et acariens, de leurs lieux de reproduction, sources de nourriture ou habitat avec une quantité à effet insecticide, nématicide ou acaricide d'un composé de formule I où X, Y, W, A, L, M et R sont tels que définis dans la revendication 1.

6. Le procédé selon la revendication 5, dans lequel le composé de formule I a la structure telle que décrite dans la revendication 2.

7. Un procédé pour protéger des plantes en croissance contre une attaque par des insectes, nématodes et acariens, comprenant l'application au feuillage desdites plantes, ou au sol ou à l'eau où elles poussent, d'une quantité à effet insecticide, nématicide ou acaricide d'un composé de formule I où X, Y, W, A, L, M et R sont tels que définis dans la revendication 1.

8. Le procédé selon la revendication 7, dans lequel ledit composé est applique auxdites plantes ou au sol dans lequel elles poussent, à une dose d'environ 0,125 kg/ha à environ 4,0 kg/ha.

9. Le procédé selon la revendication 7, dans lequel ledit composé de formule I est appliqué au feuillage desdites plantes ou au sol ou à l'eau où elles poussent sous la forme d'une composition liquide contenant environ 10 ppm à environ 10 000 ppm dudit composé de formule I.
